# DEMANDE DE BREVET EUROPEEN

(11) **EP 1 580 186 A1**
(43) Date de publication de la demande: **28.09.2005**
(21) Numéro de dépôt: 05290369.7
(22) Date de dépôt: 18.02.2005
(51) Int. Cl.: C07C 229/18, C07C 229/22, A61K 7/13

(54) **Para-phénylènediamines secondaires portant un groupement carboxy, composition tinctoriale les comprenant, procédés et utilisations**

(30) Priorité: 27.02.2004 FR 0402026
(71) Demandeur: L'OREAL, 75008 Paris (FR)
(72) Inventeur: Sabelle, Stéphane, 75005 Paris (FR); Metais, Eric, 95320 Saint-Leu-La-Foret (FR); Breton, Philippe, 78590 Noisy Le Roi (FR)
(74) Mandataire: Zapalowicz, Francis

(57) **Abrégé**

La présente demande concerne de nouvelles para-phénylènediamines secondaires portant un groupement carboxy, un procédé permettant leur préparation, leur utilisation en coloration d'oxydation capillaire, une composition pour la teinture des fibres kératiniques, et en particulier les fibres kératiniques humaines telles que les cheveux, contenant, dans un milieu approprié pour la teinture, au moins une telle para-phénylènediamine secondaire, un procédé de teinture des fibres kératiniques, l'utilisation de cette composition en coloration capillaire, ainsi qu'un "kit" de teinture.

## Description

La présente demande concerne une nouvelle famille de para-phénylènediamines secondaires portant un groupement carboxy, leur préparation et leur utilisation en la coloration d'oxydation des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

De manière surprenante et avantageuse, la Demanderesse vient de découvrir qu'il est possible d'obtenir une nouvelle famille de para-phénylènediamines secondaires portant un groupement carboxy capable de conduire à des colorations aux nuances variées, puissantes, esthétiques, peu sélectives et résistant bien aux diverses agressions que peuvent subir les fibres. La présente demande porte également sur un procédé de préparation de ces para-phénylènediamines secondaires portant un groupement carboxy, ainsi que sur leur utilisation en coloration d'oxydation capillaire.

Un autre objet de l'invention concerne des compositions pour la teinture des fibres kératiniques, en particulier des fibres kératiniques humaines telles que les cheveux, comprenant au moins une para-phénylènediamine secondaire portant un groupement carboxy permettant d'obtenir des colorations présentant les avantages précédemment mentionnés. En outre, ces compositions présentent un bon profil toxicologique.

L'invention a aussi pour objet un procédé de teinture mettant en oeuvre cette composition, l'utilisation de la composition selon la présente invention pour la teinture des fibres kératiniques, en particulier les fibres kératiniques humaines telles que les cheveux et un dispositif à plusieurs compartiments ou "kit" de teinture.

La composition de la présente invention permet en particulier d'obtenir une coloration de fibres kératiniques très puissante, peu sélective et tenace, en particulier à la lumière, tout en évitant la dégradation de ces fibres.

D'autres caractéristiques, aspects, objets et avantages de la présente invention apparaîtront encore plus clairement à la lecture de la description et des exemples qui suivent.

Les nouvelles para-phénylènediamines secondaires selon la présente invention sont des composés de formule générale (I) : 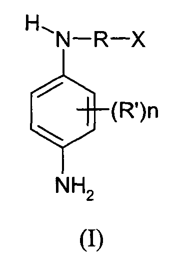 dans laquelle :
n représente un entier variant de 0 à 4,
si n varie de 1 à 4, alors R représente un radical alkylène en C₂-C₁₀ linéaire ou ramifié, non substitué ou substitué par un ou plusieurs groupements choisis parmi un hydroxy, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant être interrompu par un ou plusieurs hétéroatomes choisis parmi l'oxygène et l'azote ;
à l'exception de l'acide 2-N-(3-méthoxy-4-aminophényl)amino-éthanoïque ;
si n est égal à 0, alors R représente un radical alkylène en C₂-C₁₀ linéaire ou ramifié, non substitué ou substitué par un ou plusieurs groupements choisis parmi un hydroxy, alcoxy en C₁-C₁₅, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant être interrompu par un ou plusieurs hétéroatomes choisis parmi l'oxygène et l'azote ;
à l'exception de l'acide 6―N-(4-aminophényl)amino-caproïque ;
à l'exception de l'acide 3―N-(4-aminophényl)amino-propanoïque ;
à l'exception de l'acide 4―N-(4-aminophényl)amino-butanoïque ;
X représente un radical -COOY, dans lequel Y est choisi parmi :
- un atome d'hydrogène,
- un radical alkyle en C₁-C₁₅,
- un radical mono ou poly hydroxy alkyle en C₁-C₁₅;
R' représente un radical alkyle en C₁-C₁₅, un alcoxy en C₁-C₁₅, un hydroxy-alcoxy en C₁-C₁₅, un alcoxy(C₁-C₁₅)alkyle en C₁-C₁₅, un mono ou poly-hydroxy alkyle en C₁-C₁₅, ou un atome de chlore;
ou ses sels d'addition.

De préférence, lorsque n est égal à 0, alors le groupement R de la formule (I) représente un radical alkylène en C₂-C₁₀, tel que éthylène, propylène, butylène, pentylène, hexylène, linéaire ou ramifié non substitué ou substitué par un ou plusieurs groupements choisis parmi un alkyl(C₂-C₈)carbonyle, carboxy, alcoxy(C₂-C₈)carbonyle, ce radical alkylène pouvant être interrompu par un ou plusieurs atomes d'oxygène.

De préférence, lorsque n varie de 1 à 4, alors le groupement R de la formule (I) représente un radical alkylène en C₂-C₁₀, tel que éthylène, propylène, butylène, pentylène, hexylène, linéaire ou ramifié non substitué ou substitué par un ou plusieurs groupements choisis parmi un alkyl(C₂-C₈)carbonyle, carboxy, alcoxy(C₂-C₈)carbonyle, ce radical alkylène pouvant contenir un ou plusieurs atomes d'oxygène.

D'une manière préférée, le groupement X de la formule (I) représente un radical -COOY, dans lequel Y est choisi parmi un atome d'hydrogène, un radical alkyle en C₁-C₆, tel que méthyle, éthyle, *iso*-propyle, *tert*-butyle.

De préférence, le groupement R' de la formule (I) représente un radical alkyle en C₁-C₆, tel que méthyle, éthyle, *n*-propyle, iso-propyle, un alcoxy en C₁-C₆, un hydroxy-alcoxy en C₁-C₆, tel que β-hydroxy-éthoxy, un alcoxy(C₁-C₆)alkyle en C₁-C₆, un mono ou poly-hydroxy alkyle en C₁-C₆.

De manière plus préférentielle, les composés de formule générale (I) sont choisis parmi

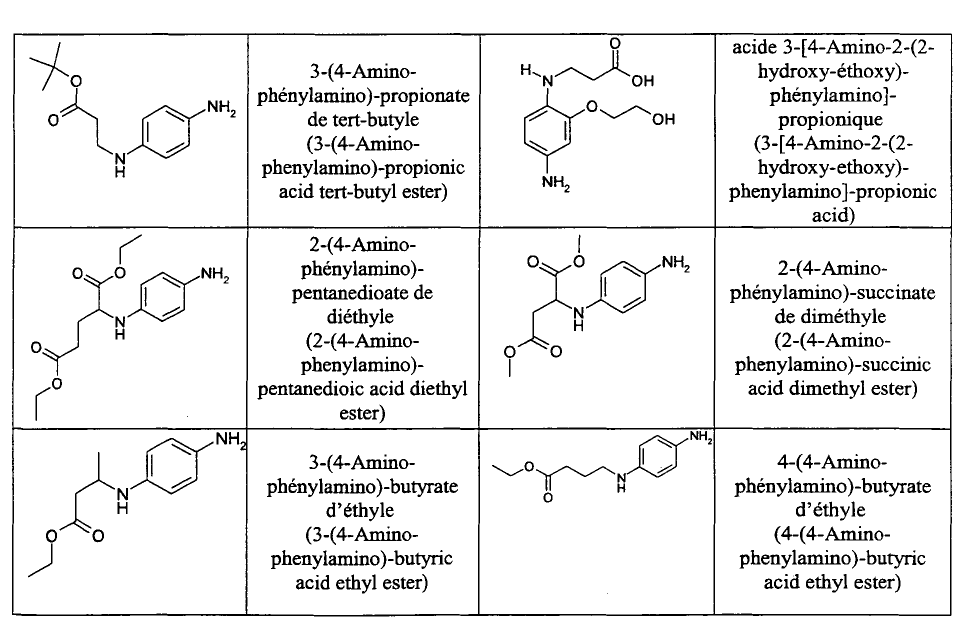

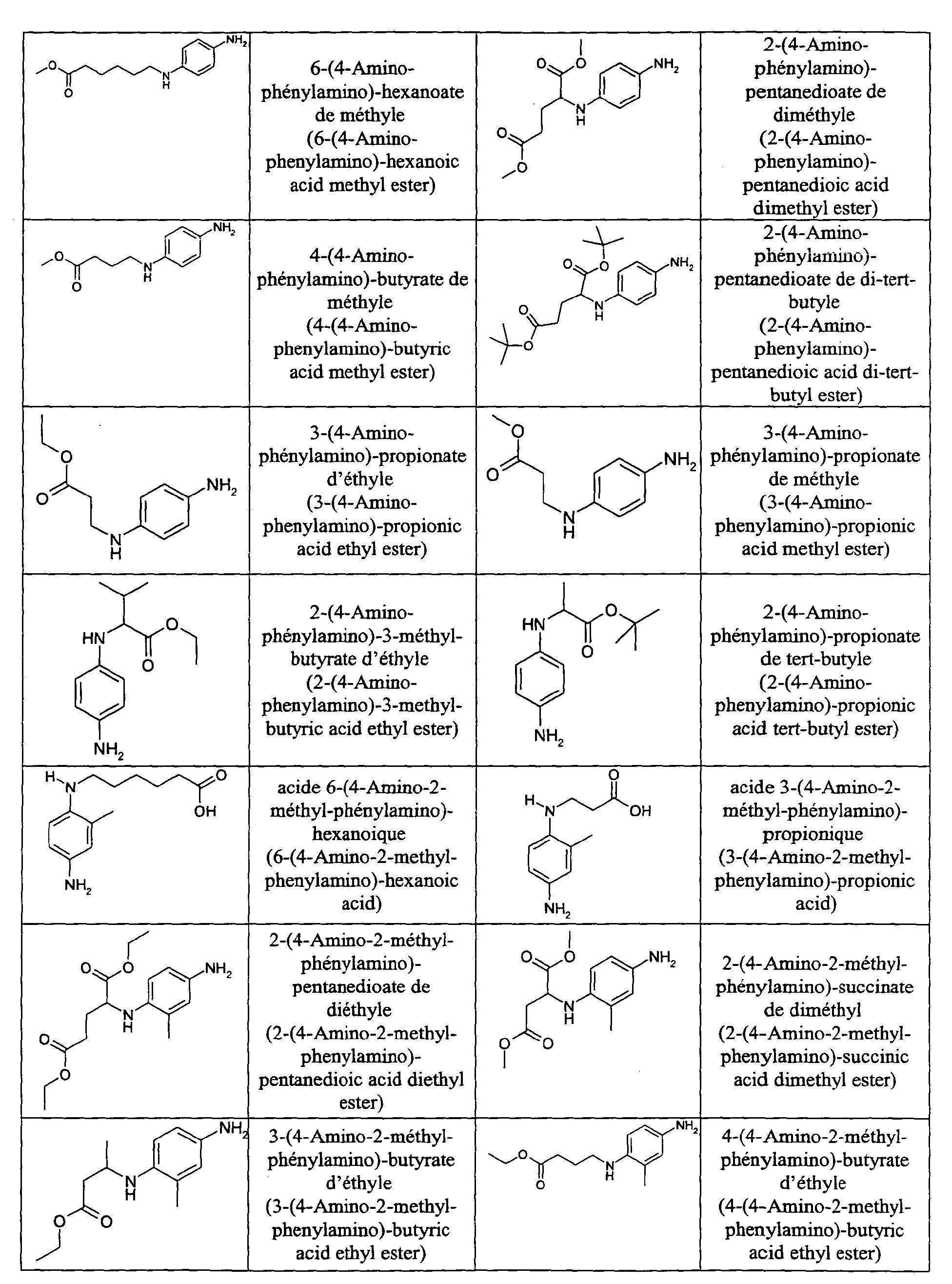

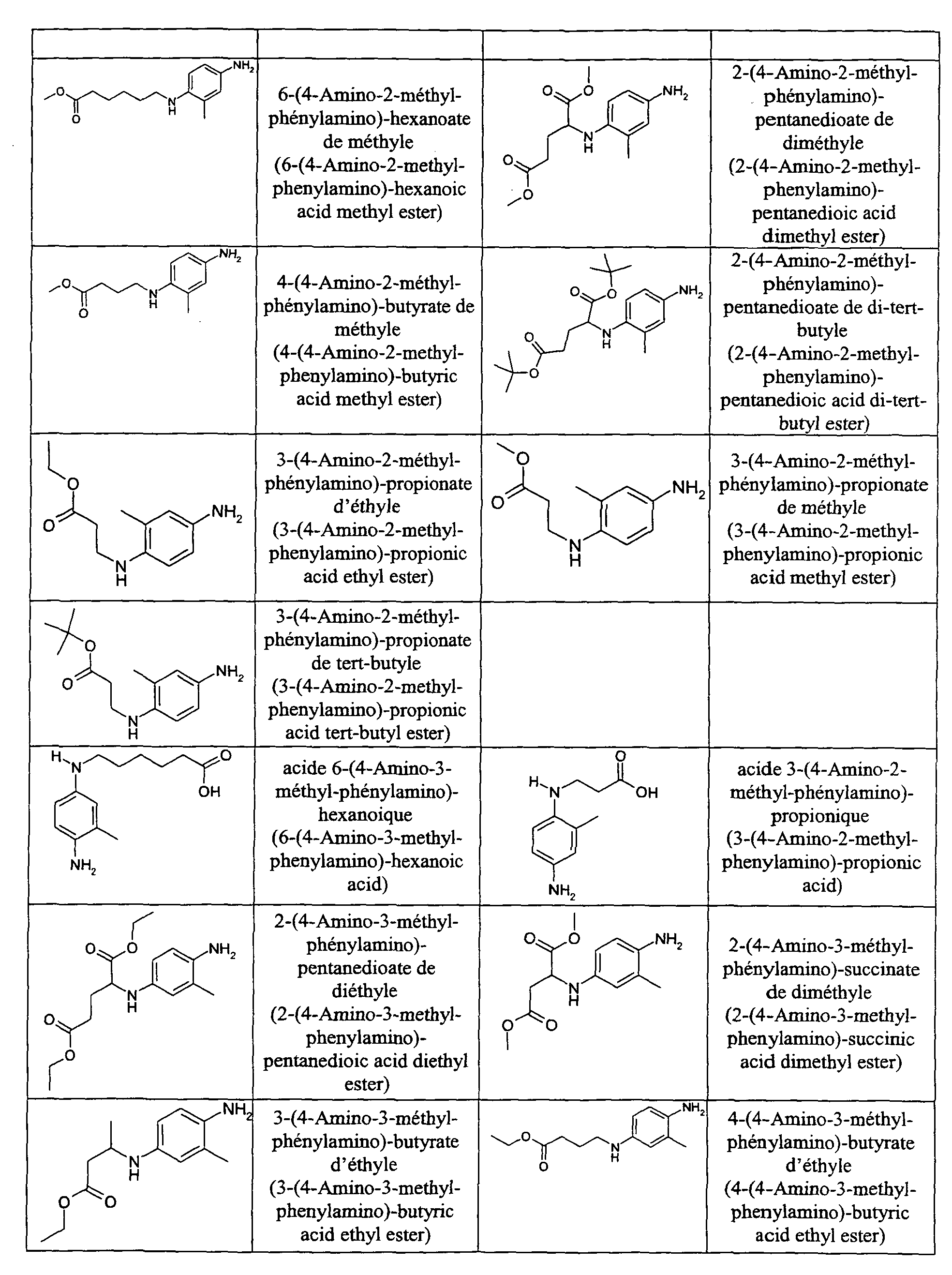

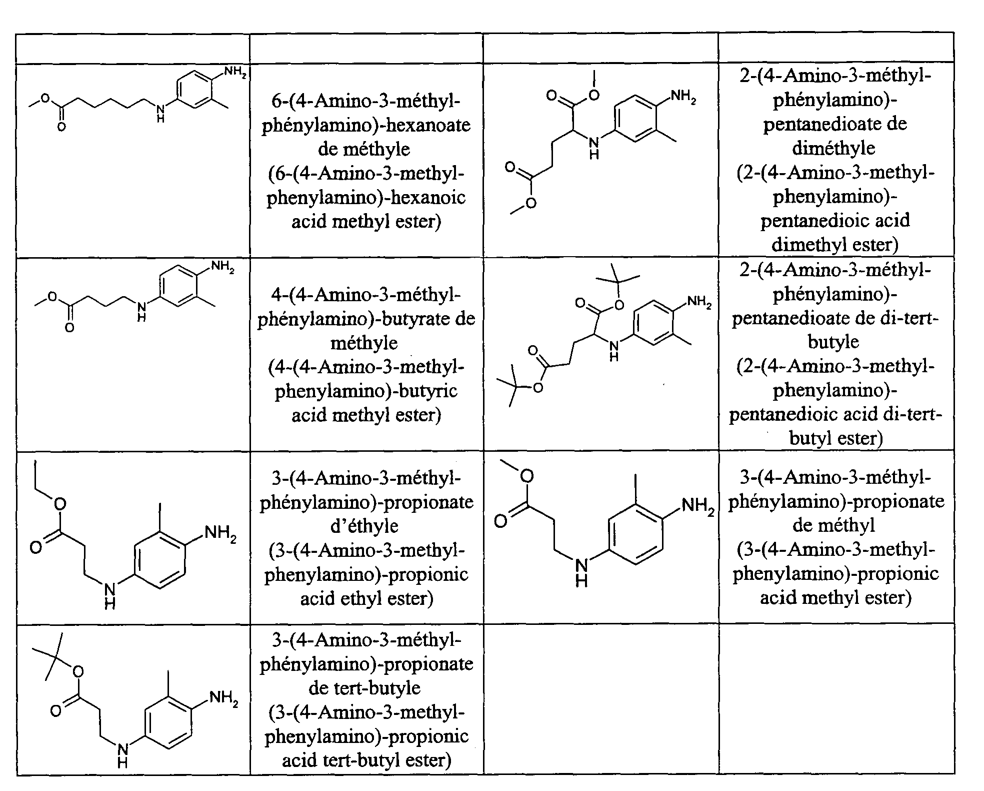

D'une manière générale, les sels d'addition sont notamment choisis parmi les sels d'addition avec un acide, tels que les chlorhydrates, les bromhydrates, les sulfates, les citrates, les succinates, les tartrates, les lactates, les tosylates, les benzènesulfonates, les phosphates et les acétates.

Les composés de formule (I) selon la présente demande peuvent être préparés de façon générale suivant une méthode qui comprend les étapes suivantes :
- substitution nucléophile de l'halogène du dérivé para-halogénonitrobenzène, par une amine primaire de formule X-RNH₂ en présence d'une base, R et X étant définis comme précédemment ;
- réduction de la fonction nitro du composé obtenu à l'étape précédente en fonction amine pour obtenir le composé de formule (I).

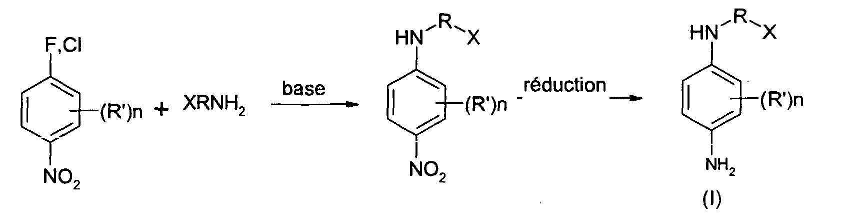

La première étape de synthèse est décrite dans les revues scientifiques Synthesis 1990 (12), 1147-1148 et Synth Commun 1990, 20(22), 3537-3545.

La dernière étape est une étape de réduction classique, par exemple en effectuant une réaction d'hydrogénation par catalyse hétérogène en présence de Pd/C, Pd(II)/C, Ni de Raney ou encore en effectuant une réaction de réduction par un métal, par exemple par du zinc, fer, étain... (Advanced Organic Chemistry, 4th edition, 1992, J. MARCH, WILEY Interscience ; Reduction in Organic Chemistry, M. Hudlicky, 1983, Ellis Honwood series Chemical Science).

Une deuxième voie de synthèse peut être schématisée comme suit :

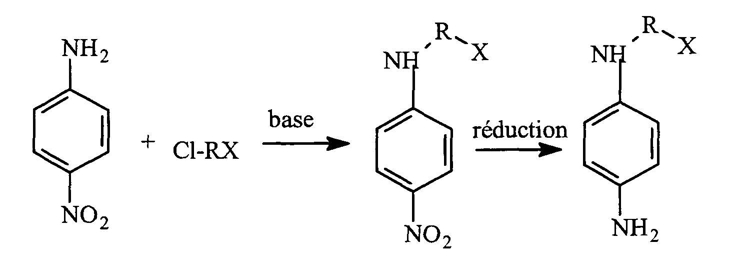

La première étape de synthèse est décrite dans les revues scientifiques J. India. Chem. Soc. 1990, 67, 602-603 et Synth Commun 1999, 29, 1819-1833.

La seconde étape est une étape de réduction identique à celle décrite ci-dessus.

La présente demande concerne aussi les composés nitrés de formule (II) 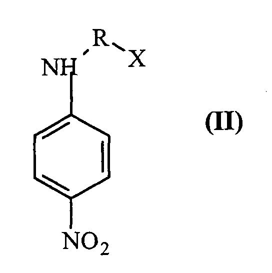 à l'exclusion de l'acide 6-N-(4-nitrophényl)aminocaproïque,
et les procédés de préparation des composés para-phénylènediamines secondaires portant un groupement carboxy de formule (I), dans lesquels on effectue une étape de réduction du composé nitré de formule (I) correspondant, le composé nitré correspondant étant le composé de formule (I) dans lequel le groupe amino en para du groupe NHR est remplacé par le groupe nitro.

La présente demande porte également sur l'utilisation du composé de formule (I) suivante pour la coloration d'oxydation capillaire : 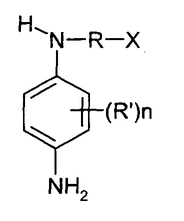 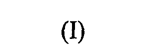 dans laquelle :
n représente un entier variant de 0 à 4,
si n varie de 1 à 4, alors R représente un radical alkylène en C₁-C₁₀ linéaire ou ramifié, non substitué ou substitué par un ou plusieurs groupements choisis parmi un hydroxy, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant contenir un ou plusieurs hétéroatomes choisis parmi l'oxygène et l'azote ;
si n est égal à 0, alors R représente un radical alkylène en C₂-C₁₀ linéaire ou ramifié, non substitué ou substitué par un ou plusieurs groupements choisis parmi un hydroxy, alcoxy en C₁-C₁₅, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant contenir un ou plusieurs hétéroatomes choisis parmi l'oxygène et l'azote ;
à l'exception de l'acide 6―N-(4-aminophényl)amino-caproïque ;
X représente un radical -COOY, dans lequel Y est choisi parmi :
- un atome d'hydrogène,
- un radical alkyle en C₁-C₁₅,
- un radical mono ou poly hydroxy alkyle en C₁-C₁₅ ;
R' représente un radical alkyle en C₁-C₁₅, un alcoxy en C₁-C₁₅, un hydroxy-alcoxy en C₁-C₁₅, un alcoxy(C₁-C₁₅)alkyle en C₁-C₁₅, un mono ou poly-hydroxy alkyle en C₁-C₁₅, ou un atome de chlore;
ou ses sels d'addition.

La présente demande concerne aussi une composition cosmétique pour la teinture des fibres, de préférence les fibres kératiniques telles que les cheveux, comprenant, dans un milieu approprié pour la teinture, au moins un composé de formule générale (I) suivante : 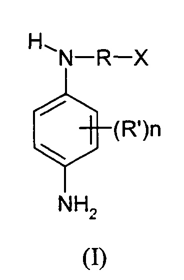 dans laquelle :
n représente un entier variant de 0 à 4,
si n varie de 1 à 4, alors R représente un radical alkylène en C₂-C₁₀ linéaire ou ramifié, non substitué ou substitué par un ou plusieurs groupements choisis parmi un hydroxy, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant contenir un ou plusieurs hétéroatomes choisis parmi l'oxygène et l'azote ;
si n est égal à 0, alors R représente un radical alkylène en C₂-C₁₀ linéaire ou ramifié, non substitué ou substitué par un ou plusieurs groupements choisis parmi un hydroxy, alcoxy en C₁-C₁₅, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant contenir un ou plusieurs hétéroatomes choisis parmi l'oxygène et l'azote ;
à l'exception de l'acide 6-N-(4-aminophényl)amino-caproïque ;
X représente un radical -COOY, dans lequel Y est choisi parmi :
- un atome d'hydrogène,
- un radical alkyle en C₁-C₁₅,
- un radical mono ou poly hydroxy alkyle en C₁-C₁₅ ;
R' représente un radical alkyle en C₁-C₁₅, un alcoxy en C₁-C₁₅, un hydroxy-alcoxy en C₁-C₁₅, un alcoxy(C₁-C₁₅)alkyle en C₁-C₁₅, un mono ou poly-hydroxy alkyle en C₁-C₁₅, ou un atome de chlore;
ou ses sels d'addition.

De préférence, lorsque n est égal à 0, alors le groupement R de la formule (I) représente un radical alkylène en C₂-C₁₀, tel que éthylène, propylène, butylène, pentylène, hexylène, linéaire ou ramifié non substitué ou substitué par un ou plusieurs groupements choisis parmi un alkyl(C₂-C₈)carbonyle, carboxy, alcoxy(C₂-C₈)carbonyle, ce radical alkylène pouvant contenir un ou plusieurs atomes d'oxygène.

De préférence, lorsque n varie de 1 à 4, alors le groupement R de la formule (I) représente un radical alkylène en C₂-C₁₀, tel que éthylène, propylène, butylène, pentylène, hexylène, linéaire ou ramifié non substitué ou substitué par un ou plusieurs groupements choisis parmi un alkyl(C₂-C₈)carbonyle, carboxy, alcoxy(C₂-C₈)carbonyle, ce radical alkylène pouvant contenir un ou plusieurs atomes d'oxygène.

D'une manière préférée, le groupement X de la formule (I) représente un radical -COOY, dans lequel Y est choisi parmi un atome d'hydrogène, un radical alkyle en C₁-C₆, tel que méthyle, éthyle, *iso*-propyle, *tert*-butyle.

De préférence, le groupement R' de la formule (I) représente un radical alkyle en C₁-C₆, tel que méthyle, éthyle, *n*-propyle, iso-propyle, un alcoxy en C₁-C₆, un hydroxy-alcoxy en C₁-C₆, tel que β-hydroxy-éthoxy, un alcoxy(C₁-C₆)alkyle en C₁-C₆, un mono ou poly-hydroxy alkyle en C₁-C₆.

De préférence, la composition contient au moins un composé de formule (I) choisi parmi les composés suivants :
- 3-(4-Amino-phénylamino)-propionate de tert-butyle ;
- acide 3-[4-Amino-2-(2-hydroxy-éthoxy)-phénylamino]-propionique ;
- 2-(4-Amino-phénylamino)-pentanedioate de diéthyle ;
- 2-(4-Amino-phénylamino)-succinate de diméthyle ;
- 3-(4-Amino-phénylamino)-butyrate d'éthyle ;
- 4-(4-Amino-phénylamino)-butyrate d'éthyle ;
- 6-(4-Amino-phénylamino)-hexanoate de méthyle ;
- 2-(4-Amino-phénylamino)-pentanedioate de diméthyle ;
- 4-(4-Amino-phénylamino)-butyrate de méthyle ;
- 2-(4-Amino-phénylamino)-pentanedioate de di-tert-butyle ;
- 3-(4-Amino-phénylamino)-propionate d'éthyle ;
- 3-(4-Amino-phénylamino)-propionate de méthyle ;
- acide 6-(4-Amino-2-méthyl-phénylamino)-hexanoique ;
- acide 3-(4-Amino-2-méthyl-phénylamino)-propionique ;
- 2-(4-Amino-2-méthyl-phénylamino)-pentanedioate de diéthyle ;
- 2-(4-Amino-2-méthyl-phénylamino)-succinate de diméthyl ;
- 3-(4-Amino-2-méthyl-phénylamino)-butyrate d'éthyl ;
- 4-(4-Amino-2-méthyl-phénylamino)-butyrate d' éthyl ;
- 6-(4-Amino-2-méthyl-phénylamino)-hexanoate de méthyl ;
- 2-(4-Amino-2-méthyl-phénylamino)-pentanedioate de diméthyle ;
- 4-(4-Amino-2-méthyl-phénylamino)-butyrate de méthyle ;
- 2-(4-Amino-2-méthyl-phénylamino)-pentanedioate de di-tert-butyle ;
- 3-(4-Amino-2-méthyl-phénylamino)-propionate d'éthyle ;
- 3-(4-Amino-2-méthyl-phénylamino)-propionate de méthyle ;
- 3-(4-Amino-2-méthyl-phénylamino)-propionate de tert-butyle ;
- 2-(4-Amino-phénylamino)-3-méthyl-butyrate d'éthyle ;
- acide 6-(4-Amino-3-méthyl-phénylamino)-hexanoique ;
- acide 3-(4-Amino-2-méthyl-phénylamino)-propionique ;
- 2-(4-Amino-3-méthyl-phénylamino)-pentanedioate de diéthyle ;
- 2-(4-Amino-3-méthyl-phénylamino)-succinate de diméthyle ;
- 3-(4-Amino-3-méthyl-phénylamino)-butyrate d'éthyle ;
- 4-(4-Amino-3-méthyl-phénylamino)-butyrate d'éthyle ;
- 6-(4-Amino-3-méthyl-phénylamino)-hexanoate de méthyle ;
- 2-(4-Amino-3-méthyl-phénylamino)-pentanedioate de diméthyle ;
- 4-(4-Amino-3-méthyl-phénylamino)-butyrate de méthyle ;
- 2-(4-Amino-3-méthyl-phénylamino)-pentanedioate de di-tert-butyle ;
- 3-(4-Amino-3-méthyl-phénylamino)-propionate d'éthyle ;
- 3-(4-Amino-3-méthyl-phénylamino)-propionate de méthyl ;
- 3-(4-Amino-3-méthyl-phénylamino)-propionate de tert-butyle ;
- 2-(4-Amino-phénylamino)-propionate de tert-butyle.

De préférence, la concentration du composé de formule générale (I) est comprise entre 0,0001 et 20%, de préférence entre 0,005 à 6% en poids par rapport au poids total de la composition.

Le milieu approprié pour la teinture est avantageusement constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

Avantageusement, la composition cosmétique comprend au moins un adjuvant cosmétique choisi dans le groupe formé par les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20 % en poids par rapport au poids de la composition.

La composition selon l'invention contient de préférence au moins un coupleur d'oxydation.

Parmi les coupleurs d'oxydation, on peut notamment citer les méta-phénylènediamines, les méta-aminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20%, de préférence entre 0,005 à 6% en poids par rapport au poids total de la composition.

La composition selon l'invention peut contenirau moins une based'oxydation additionnelle différente du composé de formule (I).

Les bases d'oxydation peuvent notamment être choisies parmi les para-phénylènediamines, les bis-phénylalkylènediamines, les para-aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4 aminophenyl pyrrolidine, le 2 thiényl para-phénylène diamine, le 2-β hydroxyéthylamino 5-amino toluène, la 3-hydroxy 1-(4' aminophenyl)pyrrolidine, la 6-(4-Amino-phenylamino)-hexan-1-ol et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6-dichlorophénol, le 4-amino-6[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation additionnelle est comprise entre 0,0001 et 20%, de préférence entre 0,005 à 6% en poids par rapport au poids total de la composition.

D'une manière générale, les sels d'addition utilisables pour les bases d'oxydation et les coupleurs sont notamment choisis parmi les sels d'addition avec un acide, tels que les chlorhydrates, les bromhydrates, les sulfates, les citrates, les succinates, les tartrates, les lactates, les tosylates, les benzènesulfonates, les phosphates et les acétates, et les sels avec une base, tels que les sels d'addition avec la soude, la potasse, l'ammoniaque, les amines ou les alcanolamines.

La composition tinctoriale conforme à l'invention peut en outre contenir un ou plusieurs colorants directs pouvant notamment être choisi parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention, on peut citer les colorants directs azoïques cationiques décrits dans les demandes de brevets WO 95/15144, WO-95/01772 et EP-714954.

Parmi ces composés, on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Bien entendu, l'homme de l'art veillera à choisir le ou les adjuvants, précurseurs de colorants d'oxydations additionnels, coupleurs d'oxydation, colorants directs de manière telle que les propriétés avantageuses attachées intrinsèquement à la composition de teinture d'oxydation conforme à l'invention ne soient pas, ou substantiellement pas, altérées par la ou les adjonctions envisagées.

Une composition tinctoriale prête à l'emploi est obtenue par ajout un ou plusieurs agents oxydants classiquement utilisés pour la teinture d'oxydation des fibres kératiniques, tels que par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, le peroxyde d'hydrogène étant particulièrement préféré, à la composition tinctoriale telle que précédemment décrite.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 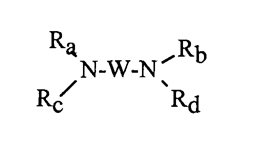 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de la présente demande concerne un procédé dans lequel on applique sur les fibres la composition telle que définie précédemment, et qu'on révèle la couleur à l'aide d'un agent oxydant. La couleur peut être révélée à pH acide, neutre ou alcalin. L'agent oxydant peut être ajouté à la composition de l'invention juste au moment de l'emploi. Il peut être mis en oeuvre à partir d'une composition oxydante le contenant, appliquée simultanément ou séquentiellement sur les fibres au moment de l'application de la composition tinctoriale.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition oxydante peut renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La présente demande concerne également l'utilisation de la composition cosmétique selon l'invention comprenant, dans un milieu approprié pour la teinture, au moins un composé de formule générale (I) pour la teinture des fibres, de préférence les fibres kératiniques telles que les cheveux.

L'invention a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### Exemple 1 : Synthèse du 3-[4-Amino-2-(2-hydroxy-éthoxy)-phénylamino]-propionic acid, dichlorhydrate (5)

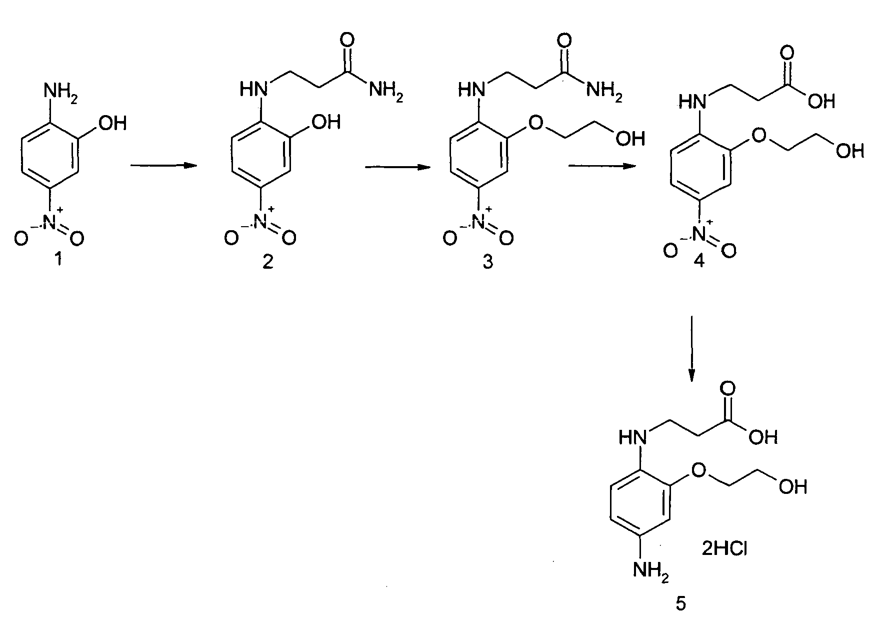

### Etape 1 : Préparation du 3-(2-hydroxy-4-nitro-phénylamino)-propionamide (2)

On porte à 100°C pendant 45 minutes 15g (0,1mole) de 2-amino-5-nitrophénol, 7,8g (0,11 mole) d'acrylamide, 3 gouttes d'acide sulfurique à 96% et 20mg d'hydroquinone. Il y a dissolution puis formation d'un insoluble que l'on filtre. Ce précipité est repris dans 100ml de toluène au reflux ; après filtration à chaud on obtient 18g de poudre après ré-empatage dans de l'acétate d'éthyle à chaud (F=194°C).

| | **théorie** | **trouvé** |
|---|---|---|
| C | 48.00 | 47.73 |
| H | 4.92 | 5.03 |
| N | 18.66 | 18.57 |

### Etape 2 : Préparation du 3-[2-(2-hydroxy-éthoxy)-4-nitro-phénylamino]-propionamide (3)

2g (8,8 mmoles) de 3-(2-hydroxy-4-nitro-phénylamino)-propionamide précédemment obtenu en solution dans 10ml de diméthylformamide et 7ml de potasse alcoolique à 15% (0,015mole) sont portés à 95°C. On ajoute à ce mélange 14ml (0,2mole) de bromo- 2-éthanol et après 2,5 heures de chauffage, on verse le mélange réactionnel sur un mélange de glace et d'eau. Le précipité jaune est essoré, lavé à la soude 1N, à l'eau puis est séché. Après recristallisation dans l'éthanol, on obtient 2,1g de poudre jaune.F=184°C.

| | **théorie** | **trouvé** |
|---|---|---|
| C | 49.07 | 48.98 |
| H | 5.62 | 5.91 |
| N | 15.61 | 14.76 |

### Etape 3 : Préparation de l'acide 3-[2-(2-Hydroxy-éthoxy)-4-nitro-phénylamino]-propionique (4)

1g de 3-[2-(2-hydroxy-éthoxy)-4-nitro-phénylamino]-propionamide (6), chauffé 1 heure au bain-marie bouillant en présence de 4ml d'acide chlorhydrique (d=1,18) et versé dans 5g de glace, donne après essorage et séchage une poudre jaune dont l'analyse élémentaire est la suivante :

| | **théorie** | **trouvé** |
|---|---|---|
| C | 48.89 | 48.04 |
| H | 5.22 | 5.85 |
| N | 10.37 | 11.03 |

### Etape 4 : Préparation du 3-[4-Amino-2-(2-hydroxy-éthoxy)-phénylamino]-propionic acid, dichlorhydrate (5)

L' acide 3-[2-(2-Hydroxy-éthoxy)-4-nitro-phénylamino]-propionique (7) obtenu dans l'étape précédente est réduit en présence de zinc/NH₄Cl pour donner l'acide 3-[4-Amino-2-(2-hydroxy-éthoxy)-phénylamino]-propionique dichlorhydraté attendu.

Les spectres RMN du proton et de masse sont conformes à la structure attendue du produit.

On obtient des mèches de cheveux colorés après application d'une composition obtenue par mélange d'un support de teinture classique comprenant le composé précédemment obtenu avec une composition oxydante comprenant du peroxyde d'hydrogène.

### Exemple 2 : Synthèse de 3-(4-Amino-phénylamino)-propionate d'éthyle, dichlorhydrate (7)

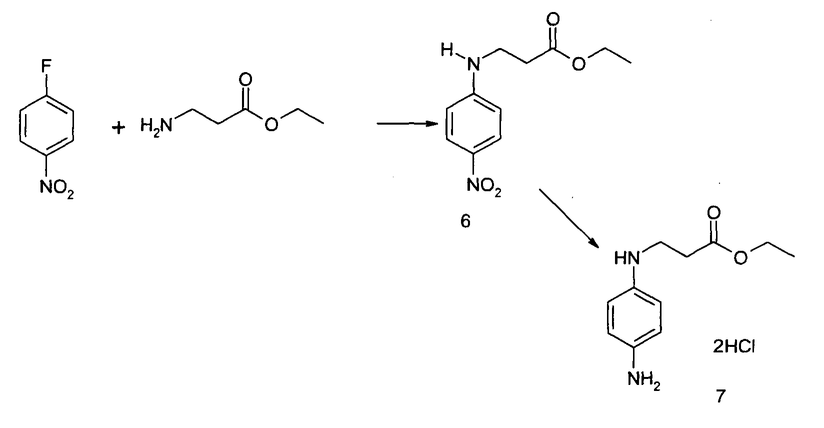

### Etape 1 : Synthèse du 3-(4-nitro-phénylamino)-propionate d' éthyle (6)

A une solution de 10 ml de N-méthyl-pyrrolidinone (NMP), on ajoute 459 mg de 4-fluoronitrobenzène, 500mg de β-alanine éthyl ester et 1,15m1 de Et₃N. Le milieu réactionnel est chauffé à 70°C pendant 8 heures, puis, après refroidissement à température ambiante, est versé dans un mélange eau + glace. Le précipité jaune formé est filtré, ré-empaté dans l'eau puis séché sur P₂O₅. On obtient 520 mg de 3-(4-nitro-phénylamino)-propionate d'éthyle (6).

### Etape 2: Synthèse du 3-(4-Amino-phénylamino)-propionate d'éthyle, dichlorhydrate (7)

Le produit obtenu lors de l'étape précédente est réduit avec un mélange zinc / chlorure d'ammonium / eau/ éthanol bouillant. L'amine correspondante est isolée sous forme de dichlorhydrate.

Les spectres RMN du proton et de masse sont conformes à la structure attendue du produit.

On obtient des mèches de cheveux colorés après application d'une composition obtenue par mélange d'un support de teinture classique comprenant le composé précédemment obtenu avec une composition oxydante comprenant du peroxyde d'hydrogène.

### Exemple 3 : Synthèse du 2-(4-Amino-phénylamino)-3-méthyl-butyrate d'éthyle, dichlorhydrate (9)

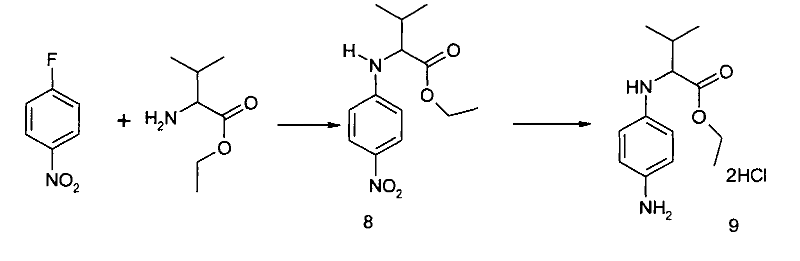

### Etape 1 : Synthèse du 2-(4-nitro-phénylamino)-3-méthyl-butyrate d'éthyle (8)

A une solution de 10 ml de N-méthyl-pyrrolidinone (NMP), on ajoute 162 mg de 4-fluoronitrobenzène, 200mg de l'ester éthylique de la L-valine et 0,4ml de Et₃N. Le milieu réactionnel est chauffé à 70°C pendant 8 heures, puis, après refroidissement à température ambiante, est versé dans un mélange eau + glace. Le précipité jaune formé est filtré, ré-empaté dans l'eau puis séché sur P₂O₅. On obtient 100mg de 2-(4-nitro-phénylamino)-3-méthyl-butyrate d'éthyle (8).

### Etape 2 : Synthèse du 2-(4-Amino-phénylamino)-3-méthyl-butyrate d'éthyle, dichlorhydrate (9)

Le composé obtenu lors de l'étape précédente est réduit avec un mélange zinc / chlorure d'ammonium / eau / éthanol bouillant. L'amine correspondante est isolée sous forme de dichlorhydrate.

Les spectres RMN du proton et de masse sont conformes à la structure attendue du produit.

On obtient des mèches de cheveux colorés après application d'une composition obtenue par mélange d'un support de teinture classique comprenant le composé précédemment obtenu avec une composition oxydante comprenant du peroxyde d'hydrogène.

### Exemple 4 : Synthèse du 2-(4-Amino-phénylamino)-propionate de tert-butyle, dichlorhydrate (11)

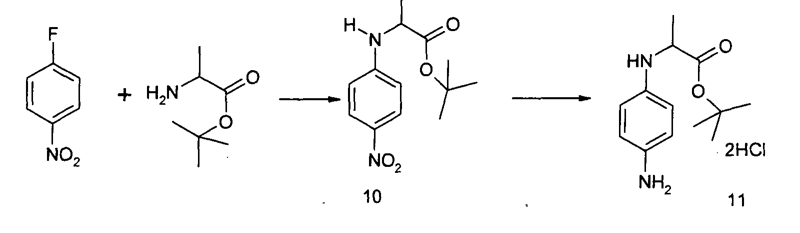

### Etape 1 : Synthèse du 2-(4-nitro-phénylamino)-propionate de tert-butyle (10)

A une solution de 10 ml de N-méthyl-pyrrolidinone (NMP), on ajoute 388 mg de 4-fluoronitrobenzène, 500mg de L-alanine terbutyl ester et 0,9ml de Et₃N. Le milieu réactionnel est chauffé à 70°C pendant 20 heures, puis, après refroidissement à température ambiante, est versé dans un mélange eau + glace. Le précipité jaune formé est filtré, ré-empaté dans l'eau puis séché sur P₂O₅. On obtient 246mg du 2-(4-nitro-phénylamino)-propionate de tert-butyle (10).

### Etape 2 : Synthèse 2-(4-Amino-phénylamino)-propionate de tert-butyle, dichlorhydrate (11)

Le composé obtenu lors de l'étape précédente est réduit avec un mélange zinc / chlorure d'ammonium / eau / éthanol bouillant. L'amine correspondant est isolée sous forme de dichlorhydrate.

On obtient des mèches de cheveux colorés après application d'une composition obtenue par mélange d'un support de teinture classique comprenant le composé précédemment obtenu avec une composition oxydante comprenant du peroxyde d'hydrogène.

### Exemple 5 : Synthèse du 3-(4-Amino-2-methyl-phenylamino)-propionic acid ethyl ester (13)

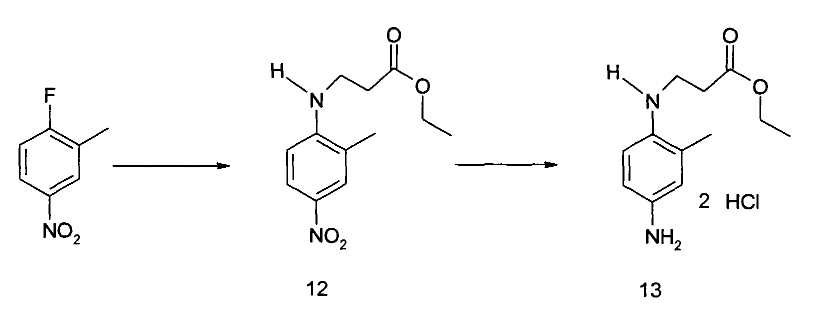

### Etape 1 : préparation du ethyl 3-[(2-methyl-4-nitrophenyl)amino]propanoate (12)

Dans un tricol, on introduit sous azote 3,1 g (0,02 mole) de 2-fluoro-5-nitrotoluène, 4,7 g (0,045 mole) de carbonate de sodium, 3,8 g (0,022 mole) de chlorhydrate d'éthyl-3-aminopropanoate et 15 ml de N-méthyl-pyrrolidinone. On chauffe jusqu'au 90 °C. Après 48 heures de réaction, le mélange réactionnel est refroidi puis 50 ml d'eau distillée sont ajoutés lentement avec forte agitation. Le dérivé nitré apparaît sous forme d'un semi-solide jaune, il est extrait au dichlorométhane puis purifié sur une colonne de silice, éluant : acétate d'éthyle / heptane : 2 / 3. On obtient 3,2 g de dérivé nitré (12) attendu sous forme d'une poudre jaune.

### Etape 2 : préparation du 3-(4-Amino-2-methyl-phenylamino)-propionic acid ethyl ester (13)

Dans un autoclave (hydrogénateur) de 200 ml, muni d'un agitateur magnétique, on introduit 2 g de dérivé nitré (12) préparé précédemment et environ 50 ml de méthanol. La solution obtenue est dégazée à l'azote. On y ajoute 0,2 g de palladium sur charbon (5% humide à 50% d'eau). On agite le mélange réactionnel, en purgeant une fois à l'hydrogène puis on introduit l'hydrogène sous pression d'environ 5 bar. Après 6 heures de réaction, le reacteur est purgé à l'azote, le mélange réactionnel est filtré rapidement sur célite sous une pression légère d'azote. Le filtrat est récupéré dans une solution préalablement refroidie de méthanol contenant 3 équivalents d'acide chlorhydrique gazeux. On rince plusieurs fois avec le méthanol sous un courant d'azote. La solution ainsi obtenue est concentrée, puis traitée avec l'éther. Le produit obtenu sous forme d'une pâte rose claire est agité puis rincé plusieurs fois avec l'acétonitrile et de l'éther sous azote. 2,3 g de produit attendu (13) est isolé sous forme d'une poudre blanche légèrement rose.

Les spectres de RMN du proton et du 13C et de microanalyses sont conformes à la structure attendue du produit.

### Exemple 6 : Synthèse du methyl 6-[(4-amino-3-methylphenyl)amino]hexanoate dihydrochloride (15)

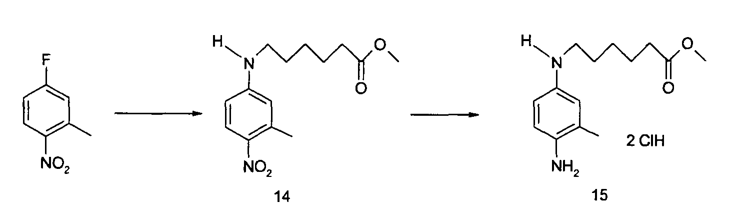

### Etape 1 : préparation du methyl 6-[(3-methyl-4-nitrophenyl)amino]hexanoate (14)

Dans un tricol, on introduit sous azote 3,1 g (0,02 mole) de 5-fluoro-2-nitrotoluène, 4,7g (0,045 mole) de carbonate de sodium, 4,5 g (0,025 mole) de chlorhydrate du méthyl-6-aminohexanoate et 20 ml de N-méthyl pyrrolidinone. On chauffe jusqu'au 90 °C. Après 56 heures de réaction, le mélange réactionnel est refroidi puis 50 ml d'eau distillée sont ajoutés lentement avec forte agitation. Un précipité jaune apparaît. Celui-ci est filtré, lavé plusieurs fois à l'eau, puis au pentane et séché sous vide. On obtient 5,3 g de dérivé nitré attendu sous forme d'une poudre jaune.

### Etape 2 : préparation du methyl 6-[(4-amino-3-methylphenyl)amino]hexanoate dihydrochloride (15)

Dans un autoclave (hydrogénateur) de 200 ml, muni d'un agitateur magnétique, on introduit 2,3 g de dérivé nitré (14) préparé précédemment et environ 100 ml de méthanol. La solution obtenue est dégazée à l'azote. On y ajoute 0,4 g de palladium sur charbon (5% humide à 50% d'eau). On agite le mélange réactionnel, en purgeant une fois à l'hydrogène puis introduit l'hydrogène sous pression d'environ 5 bar. Après 4 heures de réaction, le réateur est purgé à l'azote. Le mélange réactionnel est filtré rapidement sur célite sous une pression légère d'azote. Le filtrat est récupéré dans une solution préalablement refroidie de méthanol contenant environ 3 équivalents d'acide chlorhydrique gazeux. On rince plusieurs fois avec le méthanol sous un courant d'azote. La solution ainsi obtenue concentrée est ensuite traitée avec l'éther. Le produit obtenu sous forme d'une pâte rose claire est agité puis rincé plusieurs fois avec l'acétonitrile et de l'éther sous azote. 2,1 g de produit attendu (15 ) est isolé sous forme d'une poudre blanche légèrement rose.

Les spectres de RMN du proton et du 13C et de microanalyses sont conformes à la structure attendu du produit.

### Exemple 7 : Synthèse de l'acide 3-[(4-aminophenyl)amino]-2-hydroxypropanoïque, dichlorhydrate (16)

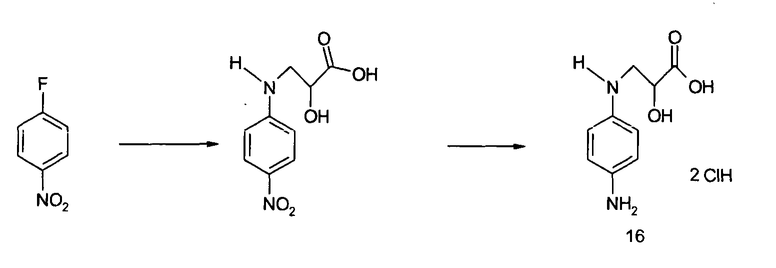

Le fluoronitrobenzène (0.2 mmol dans 400µL de N-méthyl-pyrrolidinone) et la DL-Isoserine (0.24 mmol dans 500µL de N-méthyl-pyrrolidinone) sont chauffés à 80°C pendant 24 heures en présence de carbonate de potassium (0.2mmol dans 500µL d'eau). Le milieu réactionnel est ensuite refroidi et concentré sous vide. Le résidu est repris dans 2 mL d'éthanol et le cyclohéxène (1 mL) est ajouté ainsi qu'une suspension de 50 mg de charbon palladié à 5% dans 500µL d'éthanol. Ce mélange est chauffé à 80°C pendant 3 heures. Le milieu réactionnel est alors filtré et traité par une solution d'éthanol chlorhydrique. La solution est ensuite concentrée pour fournir le dérivé attendu sous forme de dichlorhydrate.

Les spectres de RMN du proton et du 13C et de microanalyses sont conformes à la structure attendu du produit.

### Exemple 8 Synthèse de l'acide 4-[(4-aminophenyl)amino]-3-hydroxybutanoïpue, hydrochloride (17)

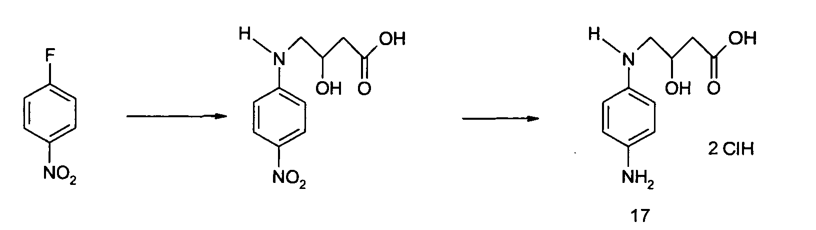

Le fluoronitrobenzène (0.2 mmol dans 400µL de N-méthyl-pyrrolidinone) et l'acide DL-4-Amino-3-hydroxybutyrique (0.24 mmol dans 500µL de N-méthyl-pyrrolidinone) sont chauffés à 80°C pendant 24 heures en présence de carbonate de potassium (0.2mmol dans 500µL d'eau). Le milieu réactionnel est ensuite refroidi et concentré sous vide. Le résidu est repris dans 2 mL d'éthanol et le cyclohéxène (1 mL) est ajouté ainsi qu'une suspension de 50 mg de charbon palladié à 5% dans 500µL d'éthanol. Ce mélange est chauffé à 80°C pendant 3 heures. Le milieu réactionnel est alors filtré et traité par une solution d'éthanol chlorhydrique. La solution est ensuite concentrée pour fournir le dérivé attendu sous forme de dichlorhydrate.

Les spectres de RMN du proton et du 13C et de microanalyses sont conformes à la structure attendue du produit.

## Revendications

1. Composé **caractérisé en ce qu'**il s'agit d'une para-phénylènediamine secondaire de formule générale (I) : 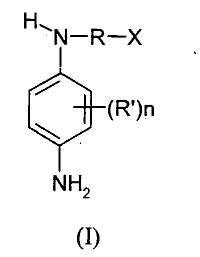 dans laquelle :
n représente un entier variant de 0 à 4,
si n varie de 1 à 4, alors R représente un radical alkylène en C₂-C₁₀ linéaire ou ramifié, non substitué ou substitué par un ou plusieurs groupements choisis parmi un hydroxy, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant contenir un ou plusieurs hétéroatomes choisis parmi l'oxygène et l'azote ;
à l'exception de l'acide 2-N-(3-méthoxy-4-aminophényl)amino-éthanoïque ;
si n est égal à 0, alors R représente un radical alkylène en C₂-C₁₀ linéaire ou ramifié, non substitué ou substitué par un ou plusieurs groupements choisis parmi un hydroxy, alcoxy en C₁-C₁₅, alkyl(C₁-C₁₅)carbonyle, alcoxy(C₁-C₁₅)carbonyle, mono ou dialkyl(C₁-C₁₅)amino carbonyle, ce radical alkylène pouvant contenir un ou plusieurs hétéroatomes choisis parmi l'oxygène et l'azote ;
à l'exception de l'acide 6-N-(4-aminophényl)amino-caproïque ;
à l'exception de l'acide 3-N-(4-aminophényl)amino-propanoïque ;
à l'exception de l'acide 4-N-(4-aminophényl)amino-butanoïque ;
X représente un radical -COOY, dans lequel Y est choisi parmi :
- un atome d'hydrogène,
- un radical alkyle en C₁-C₁₅,
- un radical mono ou poly hydroxy alkyle en C₁-C₁₅ ;
R' représente un radical alkyle en C₁-C₁₅, un alcoxy en C₁-C₁₅, un hydroxy-alcoxy en C₁-C₁₅, un alcoxy(C₁-C₁₅)alkyle en C₁-C₁₅, un mono ou poly-hydroxy alkyle en C₁-C₁₅, ou un atome de chlore;
ou ses sels d'addition.

2. Composé selon la revendication 1, **caractérisé en ce que**, lorsque n est égal à 0, le groupement R de la formule (I) représente un radical alkylène en C₂-C₁₀ linéaire ou ramifié, non substitué ou substitué par un ou plusieurs groupements choisis parmi un alkyl(C₂-C₈)carbonyle, alcoxy(C₂-C₈)carbonyle, ce radical alkylène pouvant contenir un ou plusieurs atomes d'oxygène.

3. Composé selon l'une quelconque des revendications 1 ou 2, **caractérisé en ce que**, lorsque n varie de 1 à 4, le groupement R de la formule (I) représente un radical alkylène en C₂-C₁₀ linéaire ou ramifié, non substitué ou substitué par un ou plusieurs groupements choisis parmi un alkyl(C₂-C₈)carbonyle, carboxy, alcoxy(C₂-C₈)carbonyle, ce radical alkylène pouvant contenir un ou plusieurs atomes d'oxygène.

4. Composé selon l'une quelconque des revendications 1 à 3, **caractérisé en ce que** le groupement X de la formule (I) représente un radical -COOY, dans lequel Y est choisi parmi un atome d'hydrogène, un radical alkyle en C₁-C₆.

5. Composé selon l'une quelconque des revendications 1 à 4, **caractérisé en ce que** le groupement R' de la formule (I) représente un radical alkyle en C₁-C₆, un alcoxy en C₁-C₆, un hydroxy-alcoxy en C₁-C₆, un alcoxy(C₁-C₆)alkyle en C₁-C₆, un mono ou poly-hydroxy alkyle en C₁-C₆.

6. Composé selon l'une quelconque des revendications 1 à 5, **caractérisé en ce que** le composé de formule (I) est choisi parmi les composés suivants:
- 3-(4-Amino-phénylamino)-propionate de tert-butyle ;
- acide 3-[4-Amino-2-(2-hydroxy-éthoxy)-phénylamino]-propionique ;
- 2-(4-Amino-phénylamino)-pentanedioate de diéthyle ;
- 2-(4-Amino-phénylamino)-succinate de diméthyle ;
- 3-(4-Amino-phénylamino)-butyrate d'éthyle ;
- 4-(4-Amino-phénylamino)-butyrate d'éthyle ;
- 6-(4-Amino-phénylamino)-hexanoate de méthyle ;
- 2-(4-Amino-phénylamino)-pentanedioate de diméthyle ;
- 4-(4-Amino-phénylamino)-butyrate de méthyle ;
- 2-(4-Amino-phénylamino)-pentanedioate de di-tert-butyle ;
- 3-(4-Amino-phénylamino)-propionate d'éthyle ;
- 3-(4-Amino-phénylamino)-propionate de méthyle ;
- acide 6-(4-Amino-2-méthyl-phénylamino)-hexanoique ;
- acide 3-(4-Amino-2-méthyl-phénylamino)-propionique ;
- 2-(4-Amino-2-méthyl-phénylamino)-pentanedioate de diéthyle ;
- 2-(4-Amino-2-méthyl-phénylamino)-succinate de diméthyle ;
- 3-(4-Amino-2-méthyl-phénylamino)-butyrate d'éthyle ;
- 4-(4-Amino-2-méthyl-phénylamino)-butyrate d'éthyle ;
- 6-(4-Amino-2-méthyl-phénylamino)-hexanoate de méthyle ;
- 2-(4-Amino-2-méthyl-phénylamino)-pentanedioate de diméthyle ;
- 4-(4-Amino-2-méthyl-phénylamino)-butyrate de méthyle ;
- 2-(4-Amino-2-méthyl-phénylamino)-pentanedioate de di-tert-butyle ;
- 3-(4-Amino-2-méthyl-phénylamino)-propionate d'éthyle ;
- 3-(4-Amino-2-méthyl-phénylamino)-propionate de méthyle ;
- 3-(4-Amino-2-méthyl-phénylamino)-propionate de tert-butyle ;
- 2-(4-Amino-phénylamino)-3-méthyl-butyrate d'éthyle ;
- acide 6-(4-Amino-3-méthyl-phénylamino)-hexanoique ;
- acide 3-(4-Amino-2-méthyl-phénylamino)-propionique ;
- 2-(4-Amino-3-méthyl-phénylamino)-pentanedioate de diéthyle ;
- 2-(4-Amino-3-méthyl-phénylamino)-succinate de diméthyle ;
- 3-(4-Amino-3-méthyl-phénylamino)-butyrate d'éthyle ;
- 4-(4-Amino-3-méthyl-phénylamino)-butyrate d'éthyle ;
- 6-(4-Amino-3-méthyl-phénylamino)-hexanoate de méthyle ;
- 2-(4-Amino-3-méthyl-phénylamino)-pentanedioate de diméthyle ;
- 4-(4-Amino-3-méthyl-phénylamino)-butyrate de méthyle ;
- 2-(4-Amino-3-méthyl-phénylamino)-pentanedioate de di-tert-butyle ;
- 3-(4-Amino-3-méthyl-phénylamino)-propionate d'éthyle ;
- 3-(4-Amino-3-méthyl-phénylamino)-propionate de méthyle ;
- 3-(4-Amino-3-méthyl-phénylamino)-propionate de tert-butyle ;
- 2-(4-Amino-phénylamino)-propionate de tert-butyle

7. Composé selon l'une quelconque des revendications 1 à 6, **caractérisé en ce que** les sels d'addition avec un acide des para-phénylènediamines secondaires de formule générale (I) sont choisis parmi les chlorhydrates, les bromhydrates, les sulfates, les citrates, les succinates, les tartrates, les lactates, les tosylates, les benzènesulfonates, les phosphates et les acétates.

8. Composé nitré **caractérisé en ce qu'**il possède la structure suivante (II) : 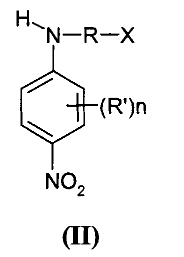 dans laquelle :
n représente un entier variant de 0 à 4,
si n varie de 1 à 4, alors R représente un radical alkylène en C₂-C₁₀ linéaire ou ramifié, non substitué ou substitué par un ou plusieurs groupements choisis parmi un hydroxy, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant être interrompu par un ou plusieurs hétéroatomes choisis parmi l'oxygène et l'azote ;
si n est égal à 0, alors R représente un radical alkylène en C₂-C₁₀ linéaire ou ramifié, non substitué ou substitué par un ou plusieurs groupements choisis parmi un hydroxy, alcoxy en C₁-C₁₅, alkyl(C₁-C₁₅)carbonyle, alcoxy(C₁-C₁₅)carbonyle, mono ou dialkyl(C₁-C₁₅)amino carbonyle, ce radical alkylène pouvant être interrompu par un ou plusieurs hétéroatomes choisis parmi l'oxygène et l'azote ;
X représente un radical -COOY, dans lequel Y est choisi parmi :
- un atome d'hydrogène,
- un radical alkyle en C₁-C₁₅,
- un radical mono ou poly hydroxy alkyle en C₁-C₁₅ ;
R' représente un radical alkyle en C₁-C₁₅, un alcoxy en C₁-C₁₅, un hydroxy-alcoxy en C₁-C₁₅, un alcoxy(C₁-C₁₅)alkyle en C₁-C₁₅, un mono ou poly-hydroxy alkyle en C₁-C₁₅, ou un atome de chlore
A l'exclusion de l'acide 6-N-(4-nitrophényl)aminocaproïque.

9. Procédé de préparation du composé de formule (I) tel que défini dans les revendications 1 à 7, **caractérisé par le fait qu'**on effectue une étape de réduction d'un composé nitré correspondant au composé de formule (I).

10. Utilisation du composé de formule (I) suivante : 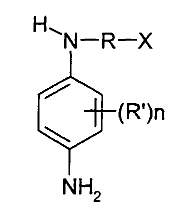 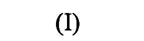 dans laquelle :
n représente un entier variant de 0 à 4,
si n varie de 1 à 4, alors R représente un radical alkylène en C₂-C₁₀ linéaire ou ramifié, non substitué ou substitué par un ou plusieurs groupements choisis parmi un hydroxy, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant contenir un ou plusieurs hétéroatomes choisi parmi l'oxygène et l'azote ;
si n est égal à 0, alors R représente un radical alkylène en C₂-C₁₀ linéaire ou ramifié, non substitué ou substitué par un ou plusieurs groupements choisis parmi un hydroxy, alcoxy en C₁-C₁₅, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant contenir un ou plusieurs hétéroatomes choisi parmi l'oxygène et l'azote ;
à l'exception de l'acide 6―N-(4-aminophényl)amino-caproïque ;
X représente un radical -COOY, dans lequel Y est choisi parmi :
- un atome d'hydrogène,
- un radical alkyle en C₁-C₁₅,
- un radical mono ou poly hydroxy alkyle en C₁-C₁₅ ;
R' représente un radical alkyle en C₁-C₁₅, un alcoxy en C₁-C₁₅, un hydroxy-alcoxy en C₁-C₁₅, un alcoxy(C₁-C₁₅)alkyle en C₁-C₁₅, un mono ou poly-hydroxy alkyle en C₁-C₁₅, ou un atome de chlore;
ou ses sels d'addition, pour la coloration d'oxydation capillaire.

11. Composition cosmétique pour la teinture des fibres kératiniques, de préférence les fibres kératiniques humaines, telles que les cheveux, comprenant, dans un milieu approprié pour la teinture, au moins un composé de formule générale (I) : 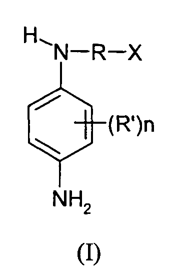 dans laquelle :
n représente un entier variant de 0 à 4,
si n varie de 1 à 4, alors R représente un radical alkylène en C₂-C₁₀ linéaire ou ramifié, non substitué ou substitué par un ou plusieurs groupements choisis parmi un hydroxy, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant contenir un ou plusieurs hétéroatomes choisi parmi l'oxygène et l'azote ;
si n est égal à 0, alors R représente un radical alkylène en C₂-C₁₀ linéaire ou ramifié, non substitué ou substitué par un ou plusieurs groupement choisi parmi un hydroxy, alcoxy en C₁-C₁₅, alkyl(C₁-C₁₅)carbonyle, carboxy, amido, alcoxy(C₁-C₁₅)carbonyle, mono ou dialkyl(C₁-C₁₅)amino carbonyle, ce radical alkylène pouvant contenir un ou plusieurs hétéroatomes choisis parmi l'oxygène et l'azote ;
à l'exception de l'acide 6―N-(4-aminophényl)amino-caproïque ;
X représente un radical -COOY, dans lequel Y est choisi parmi :
- un atome d'hydrogène,
- un radical alkyle en C₁-C₁₅,
- un radical mono ou poly hydroxy alkyle en C₁-C₁₅ ;
R' représente un radical alkyle en C₁-C₁₅, un alcoxy en C₁-C₁₅, un hydroxy-alcoxy en C₁-C₁₅, un alcoxy(C₁-C₁₅)alkyle en C₁-C₁₅, un mono ou poly-hydroxy alkyle en C₁-C₁₅, ou un atome de chlore;
ou ses sels d'addition.

12. Composition selon la revendication 11, **caractérisée en ce que**, lorsque n est égal à 0, le groupement R de la formule (I) représente un radical alkylène en C₂-C₁₀ linéaire ou ramifié non substitué ou substitué par un ou plusieurs groupements choisis parmi un alkyl(C₂-C₈)carbonyle, carboxy, alcoxy(C₂-C₈)carbonyle, ce radical alkylène pouvant contenir un ou plusieurs atomes d'oxygène.

13. Composition selon l'une quelconque des revendications 11 ou 12, **caractérisée en ce que**, lorsque n varie de 1 à 4, le groupement R de la formule (I) représente un radical alkylène en C₂-C₁₀, linéaire ou ramifié non substitué ou substitué par un ou plusieurs groupements choisis parmi un alkyl(C₂-C₈)carbonyle, carboxy, alcoxy(C₂-C₈)carbonyle, ce radical alkylène pouvant contenir un ou plusieurs atomes d'oxygène.

14. Composition selon l'une quelconque des revendications 11 à 13, **caractérisée en ce que** le groupement X de la formule (I) représente un radical -COOY, dans lequel Y est choisi parmi un atome d'hydrogène, un radical alkyle en C₁-C₆.

15. Composition selon l'une quelconque des revendications 11 à 14, **caractérisée en ce que** le groupement R' de la formule (I) représente un radical alkyle en C₁-C₆, un alcoxy en C₁-C₆, un hydroxy-alcoxy en C₁-C₆, un alcoxy(C₁-C₆)alkyle en C₁-C₆, un mono ou poly-hydroxy alkyle en C₁-C₆.

16. Composition selon l'une quelconque des revendications 11 à 15, **caractérisée en ce que** le composé de formule (I) est choisi parmi les composés suivants :
- 3-(4-Amino-phénylamino)-propionate de tert-butyle ;
- acide 3-[4-Amino-2-(2-hydroxy-éthoxy)-phénylamino]-propionique ;
- 2-(4-Amino-phénylamino)-pentanedioate de diéthyle ;
- 2-(4-Amino-phénylamino)-succinate de diméthyle ;
- 3-(4-Amino-phénylamino)-butyrate d'éthyle ;
- 4-(4-Amino-phénylamino)-butyrate d'éthyle ;
- 6-(4-Amino-phénylamino)-hexanoate de méthyle ;
- 2-(4-Amino-phénylamino)-pentanedioate de diméthyle ;
- 4-(4-Amino-phénylamino)-butyrate de méthyle ;
- 2-(4-Amino-phénylamino)-pentanedioate de di-tert-butyle ;
- 3-(4-Amino-phénylamino)-propionate d'éthyle ;
- 3-(4-Amino-phénylamino)-propionate de méthyle ;
- acide 6-(4-Amino-2-méthyl-phénylamino)-hexanoique ;
- acide 3-(4-Amino-2-méthyl-phénylamino)-propionique ;
- 2-(4-Amino-2-méthyl-phénylamino)-pentanedioate de diéthyle ;
- 2-(4-Amino-2-méthyl-phénylamino)-succinate de diméthyle ;
- 3-(4-Amino-2-méthyl-phénylamino)-butyrate d'éthyle ;
- 4-(4-Amino-2-méthyl-phénylamino)-butyrate d'éthyle ;
- 6-(4-Amino-2-méthyl-phénylamino)-hexanoate de méthyle ;
- 2-(4-Amino-2-méthyl-phénylamino)-pentanedioate de diméthyle ;
- 4-(4-Amino-2-méthyl-phénylamino)-butyrate de méthyle ;
- 2-(4-Amino-2-méthyl-phénylamino)-pentanedioate de di-tert-butyle ;
- 3-(4-Amino-2-méthyl-phénylamino)-propionate d'éthyle ;
- 3-(4-Amino-2-méthyl-phénylamino)-propionate de méthyle ;
- 3-(4-Amino-2-méthyl-phénylamino)-propionate de tert-butyle ;
- 2-(4-Amino-phénylamino)-3-méthyl-butyrate d'éthyle ;
- acide 6-(4-Amino-3-méthyl-phénylamino)-hexanoique ;
- acide 3-(4-Amino-2-méthyl-phénylamino)-propionique ;
- 2-(4-Amino-3-méthyl-phénylamino)-pentanedioate de diéthyle ;
- 2-(4-Amino-3-méthyl-phénylamino)-succinate de diméthyle ;
- 3-(4-Amino-3-méthyl-phénylamino)-butyrate d'éthyle ;
- 4-(4-Amino-3-méthyl-phénylamino)-butyrate d'éthyle ;
- 6-(4-Amino-3-méthyl-phénylamino)-hexanoate de méthyle ;
- 2-(4-Amino-3-méthyl-phénylamino)-pentanedioate de diméthyle ;
- 4-(4-Amino-3-méthyl-phénylamino)-butyrate de méthyle ;
- 2-(4-Amino-3-méthyl-phénylamino)-pentanedioate de di-tert-butyle ;
- 3-(4-Amino-3-méthyl-phénylamino)-propionate d'éthyle ;
- 3-(4-Amino-3-méthyl-phénylamino)-propionate de méthyle ;
- 3-(4-Amino-3-méthyl-phénylamino)-propionate de tert-butyle ;
- 2-(4-Amino-phénylamino)-propionate de tert-butyle.

17. Composition selon l'une quelconque des revendications 11 à 16, **caractérisée en ce que** la concentration du composé de formule générale (I) est comprise entre 0,0001 et 20%, de préférence entre 0,005 à 6% en poids par rapport au poids total de la composition.

18. Composition selon l'une quelconque des revendications 11 à 17, **caractérisée en ce que** le milieu approprié pour la teinture est constitué par de l'eau ou par un mélange d'eau et d'au moins un solvant organique choisi parmi les alcools inférieurs en C₁-C₄, ramifiés ou non; les polyols et éthers de polyols; les alcools aromatiques et leurs mélanges.

19. Composition selon l'une quelconque des revendications 11 à 18, **caractérisée en ce qu'**elle comprend au moins un adjuvant cosmétique choisi parmi les agents anti-oxydants, les agents de pénétration, les agents séquestrants, les parfums, les tampons, les agents dispersants, les tensioactifs, les agents de conditionnement, les agents filmogènes, les polymères, les céramides, les agents conservateurs, les agents nacrants ou opacifiants, les vitamines ou provitamines.

20. Composition selon la revendication 19, **caractérisée en ce que** la quantité en adjuvant cosmétique est comprise pour chacun d'eux entre 0,01 et 20 % en poids par rapport au poids total de la composition.

21. Composition selon l'une quelconque des revendications 11 à 20, **caractérisée en ce que** la composition comprend au moins un coupleur d'oxydation choisi parmi les méta-phénylènediamines, les méta-aminophénols, les méta--diphénols, les coupleurs naphtaléniques, les coupleurs hétérocycliques ainsi que leurs sels d'addition.

22. Composition selon la revendication 21, **caractérisée en ce que** la concentration du ou des coupleurs est comprise entre 0,0001 et 20 % en poids par rapport au poids total de la composition.

23. Composition selon l'une quelconque des revendications 11 à 22, **caractérisée en ce que** la composition comprend au moins une base d'oxydation additionnelle différente de celle de formule (I) choisie parmi les para-phénylènediamines, les bis-phénylalkylènediamines, les para-aminophénols, les ortho-aminophénols, les bases hétérocycliques et leurs sels d'addition.

24. Composition selon la revendication 23, **caractérisée en ce que** la concentration de la ou des bases d'oxydation additionnelle est comprise entre 0,0001 et 20% en poids par rapport au poids total de la composition.

25. Composition selon l'une quelconque des revendications 11 à 24, **caractérisée en ce qu'**elle renferme un ou plusieurs colorants directs naturels ou cationiques.

26. Procédé de teinture des fibres kératiniques et en particulier des fibres kératiniques humaines telles que les cheveux, **caractérisé par le fait qu'**on applique sur lesdites fibres au moins une composition selon l'une quelconque des revendications 11 à 25, pendant une durée suffisante pour développer la coloration désirée en présence d'un agent oxydant.

27. Composition prête à l'emploi, **caractérisée en ce qu'**elle comprend dans une composition tinctoriale telle que définie dans l'une quelconque des revendications 11 à 25 au moins un agent oxydant choisi parmi le peroxyde d'hydrogène, le peroxyde d'urée, les bromates de métaux alcalins, les persels, les peracides et les enzymes oxydases, et de préférence le peroxyde d'hydrogène

28. Utilisation pour la teinture des fibres kératiniques et en particulier des fibres kératiniques humaines telles que les cheveux de la composition telle que définie dans l'une quelconque des revendications 11 à 25.

29. Dispositif à plusieurs compartiments dans lequel le premier compartiment contient une composition tinctoriale pour la teinture des fibres kératiniques telle que définie à l'une quelconque des revendications 11 à 25 et un deuxième compartiment contient un agent oxydant.
